# EUROPEAN PATENT APPLICATION

(11) **EP 4 296 667 A1**
(43) Date of publication of application: **27.12.2023**
(21) Application number: 22189381.1
(22) Date of filing: 09.08.2022
(51) Int. Cl.: G01N 33/14, G01N 21/51, G01N 15/02

(54) **JUICE FRESHNESS MONITORING METHOD AND SYSTEM**

(30) Priority: 23.06.2022 WO PCT/CN2022/100887
(71) Applicant: Versuni Holding B.V., 5656 AE Eindhoven (NL)
(72) Inventor: CHEN, Yun, 5656 AE Eindhoven (NL)
(74) Representative: Vollering, Stefanus Franciscus Maria

(57) **Abstract**

Provided is a method (100) for communicating data relevant to a freshness of juice stored in a portable container. The method comprises receiving (102) optical data from an optical sensor arranged to sense particles in the juice stored in the portable container. The determination (104) of the data relevant to the freshness of the juice stored in the portable container comprises extracting a measure of particle agglomeration in the juice from the optical data. The thus determined data is then communicated (106) to a user of the portable container. Further provided is a computer program for implementing the method, and a related system for communicating the data relevant to the freshness of the juice stored in the portable container.

## Description

### FIELD OF THE INVENTION

The invention relates to a method of communicating data relevant to a freshness of juice stored in a portable container, and a computer program for implementing the method. The invention also provides a system for communicating such data relevant to the freshness of juice stored in a portable container.

### BACKGROUND OF THE INVENTION

Many consumers make use of juicers and/or blenders to assist them in consuming healthy food types, such as vegetables and fruits, which are rich in nutrients, such as dietary fibre, vitamins, and minerals.

Consumers' concerns about processed food can mean that they desire the food they are consuming to be as close as possible to raw ingredients. Freshness is a key attribute and an indicator of goodness.

Whilst consumers enjoy the freshness of healthy fruit and vegetable drinks, such drinks tend not to stay fresh for long. Juices can lose taste, colour and separate relatively quickly after preparation.

There are currently several solutions on the market aiming to prolong the freshness of fruit and vegetable drinks. For example, so-called vacuum blenders are available that provide oxygenfree conditions for smoothie making. When using such a device, micronutrients that are sensitive to oxygen can be preserved.

Whilst offering a promising approach for preserving freshness, the manner in which the drinks prepared in the vacuum blender is stored after blending can present a problem. This is because when the vacuum blender is opened after blending, the smoothie is exposed to environmental conditions, for instance light and oxygen, and oxidation can occur. The freshness of the drink is consequently affected.

Portable blenders offer an alternative approach to providing the consumer with fresh fruit and vegetable drinks. However, a disadvantage of such portable blenders is that the consumer is required to prepare ingredients, in particular by cutting the ingredients into pieces, in advance. When the consumer is on the move, such pre-cut ingredients risk being oxidized prior to being blended. Moreover, such portable blenders may not offer an efficient way to preserve the freshness of the drinks once they have been blended. For this reason, immediate consumption of the drink prepared using the portable blender is recommended.

Also known are sensors and indicators used in smart food packaging for tracking the freshness of foods, in particular tracking the freshness of foods in the supply chain. Such sensors tend to be mainly used for monitoring the freshness of meat, seafood, frozen foods, milk-based products and fresh fruits. Common indicators/sensors include time and temperature indicators (TTI) which are relatively widely used to monitor quality by tracking the temperature conditions to which foodstuffs have been exposed over time; freshness indicators which directly indicate the quality of the product by detecting volatile metabolites linked to the metabolism of microorganisms, such as amines, organic acids, glucose and so on; gas sensors used for tracking leakage within packaging and monitoring the spoilage progress via measurement of the concentration of certain gases, such as O₂, CO₂ or H₂S; biosensors focusing on tracking and monitoring foodborne pathogens; and electronic nose and tongue sensing systems that, whilst not yet fully commercialized, employ taste and smell sensors for sensing freshness and quality of foods.

The above-described sensors and indicators may not necessarily be suitable or convenient for providing information to a consumer concerning the status of a fruit or vegetable drink they have prepared themselves under domestic/household conditions.

Whilst smoothies prepared, in other words blended, under such domestic/household conditions, may be relatively stable with respect of separation, this tends not to be the case for clear juices. Separation of initially clear juices may be particularly undesirable from the consumers' point of view, but this may occur, as well as other undesirable phenomena taking place during juice storage, without the consumer being adequately informed, warned or advised to consume the stored juice before separation takes place or to discard it due to poor quality.

### SUMMARY OF THE INVENTION

The invention is defined by the claims.

According to examples in accordance with an aspect of the invention, there is provided a method for communicating data relevant to a freshness of juice stored in a portable container, the method comprising: receiving optical data from an optical sensor arranged to sense particles in the juice stored in the portable container; determining the data relevant to the freshness of the juice stored in the portable container, wherein the determining comprises extracting a measure of particle agglomeration in the juice from the optical data; and communicating the data to a user of the portable container.

The present disclosure is based, at least in part, on the insight that the agglomeration behavior of particles in juice, for example juice extracted from fruit and/or vegetables, can be used as a convenient freshness indicator of the juice when stored in a portable container. This is because the timescale of the agglomeration may align with the timescale of decomposition processes, for example oxidation, associated with declining freshness. Moreover, a measure of particle agglomeration in the stored juice can be conveniently extracted from optical data received from an optical sensor arranged to sense particles in the juice stored in the portable container. Such an optical sensor can, for instance, be mounted in and/or on the portable container.

Any suitable optical sensor can be used provided that the optical sensor is capable of sensing particles in the juice and generating optical data from which the measure of particle agglomeration can be extracted.

In some embodiments, the optical sensor comprises an image detection unit, for example a camera.

Such an image detection unit may include an image sensor, for example a charge coupled device (CCD) image sensor or a complementary metal-oxide-semiconductor (CMOS) image sensor.

In some embodiments, the image detection unit comprises an optical microscope that cooperates with the image sensor to allow microscope images to be captured.

In such embodiments, the image sensor and the optical microscope may be conveniently provided as an integrated unit or as separate units in which the image sensor is arranged to capture a microscope image of the juice provided by the optical microscope.

An image detection unit may provide image data, for example a characteristic of one or more particle agglomerates in the juice, from which the measure of particle agglomeration is extractable.

In general, the optical sensor may, via sensing of the particles in the juice, generate the optical data in a first step, and an optical data processing module, e.g. in the form of an image processing module, included in a processing system may, in a second step, extract the measure of particle agglomeration in the stored juice from the optical data.

The first step may, for instance, comprise the image sensor(s) of the image detection unit capturing a real-time image of the juice, with the second step comprising (i) analyzing and extracting a particle size distribution using an algorithm, e.g. a backend algorithm, run on the processing system, and (ii) determining an average particle size from the particle size distribution.

The average particle size may, for instance, be a median particle size, in other words D₅₀, obtained from the particle size distribution.

The measure of particle agglomeration may be extracted from optical data, e.g. image data enabling assessment of particle assemblage(s) in the juice, captured at a single point in time, or may be extracted from optical data, e.g. image data, captured at a plurality of points over a period time.

As an example of extracting the measure of particle agglomeration from optical data captured at a single point in time, the average particle size, e.g. D₅₀, can be determined from an image of the juice in the manner described above, a comparison of the thus determined average particle size with a look-up table can be made, with the comparison enabling extraction of the measure of particle agglomeration in the juice. The data relevant to the freshness of the juice stored in the portable container can then be communicated to the user, for example immediately following the determination of the data, e.g. in the form of a real-time message.

In some embodiments, the measure of particle agglomeration comprises a measure of change in one or more parameters of a particle size distribution over a juice storage period.

The particle agglomeration may cause a particle size distribution to change over a juice storage period, in other words as a function of time during which the juice is stored in the portable container. This may be due to growing particle assemblages increasing the relative amount of larger particles evident in the particle size distribution.

In some embodiments, the one or more parameters of the particle size distribution is or are selected from an average particle size, e.g. D₅₀, determined from the particle size distribution and a dispersity of the particle size distribution.

In some embodiments, the optical data comprises initial optical data obtained by the optical sensor at a time of the juice being initially stored in the portable container, and subsequent optical data obtained by the optical sensor after one or more time periods from the time of the juice being initially stored, with the determining the data relevant to the freshness of the juice stored in the portable container comprising extracting the measure of particle agglomeration by comparison of the initial optical data and the subsequent optical data.

Such a comparison between the initial optical data and the subsequent optical data may assist in terms of permitting the freshness of the juice following the time period(s) to be compared to the freshness of the juice upon being initially stored in the portable container. In this way, the measure of particle agglomeration can be made independent of the particle size in the asprepared juice.

In some embodiments, the one or more time periods each have a predetermined duration.

Thus, the measure of particle agglomeration can be obtained at one or more defined points in time during storage of the juice. This can assist with freshness monitoring.

In some embodiments, the method comprises controlling the optical sensor to obtain the subsequent optical data after the one or more time periods from the time of the juice being initially stored.

The controlling the optical sensor to obtain the subsequent optical data may be implemented automatically.

This may assist to alleviate the burden on the user to him/herself take steps to monitor the freshness of the juice stored in the portable container.

In alternative embodiments, the user may him/herself control the optical sensor to obtain the subsequent optical data, for example in response to a prompt provided via a user interface, or unprompted, e.g. when considering whether to drink the juice stored in the portable container.

In some embodiments, the data relevant to the freshness of the juice stored in the portable container comprises a sediment status indicator indicative of a degree of sedimentation in the juice.

Thus, the determining may include determining the sediment status indicator based on the measure of agglomeration extracted from the optical data.

In some embodiments, the sediment status indicator is a sediment level selected from a plurality of pre-set levels.

By way of a non-limiting example, such a plurality of levels may include or be defined by the following: Level 0: fresh as 'just-made' and no detectable sediment; Level 1: drinkable but slight sedimentation detected; Level 2: drinkable but moderate sedimentation detected; and Level 3: suggest not to drink.

In such an example, Level 0 may be based on an average particle size in the stored juice being <109 µm; Level 1 may be based on an average particle size in the stored juice being 110-159 µm; Level 2 may be based on an average particle size in the stored juice being 160-600 µm; and Level 3 may be based on an average particle size in the stored juice being >600 µm.

More generally, the data relevant to the freshness of the juice stored in the portable container may be communicated, for example as a report and/or reminder message to the user, to enable the user to decide whether or not to drink the stored juice.

In some embodiments, the data relevant to the freshness of the juice stored in the portable container comprises a reminder for the user to consume the juice and/or an estimated time left for the user to consume the juice.

Thus, the determining may include determining the reminder and/or the estimated time left for the user to consume the juice based on the measure of agglomeration extracted from the optical data.

Such a reminder and/or estimated time left may assist the user to consume the stored juice while it is still sufficiently fresh.

In at least some embodiments, the communicating the data relevant to the freshness of the juice stored in the portable container comprises controlling a user interface to communicate the data.

Such a user interface may include a display and/or a speaker, such that the data may be communicated visually and/or audibly.

In some embodiments, the communicating the data relevant to the freshness of the juice stored in the portable container comprises controlling a display of a portable device to display the data.

Such a portable device may enable the user to receive the data while on the move, e.g. when on the move with the portable container. The portable device may, for instance, be a smartphone and/or a tablet computer.

According to another aspect there is provided a computer program product comprising computer program code which, when executed on a computing device having a processing system, causes the processing system to perform the method according to any of the embodiments described herein.

One or more non-transitory computer readable media may be provided, which non-transitory computer readable media have a computer program stored thereon, with the computer program comprising computer program code which is configured, when the computer program is run on a processing system, to cause the processing system to implement the method according to any of the embodiments described herein.

According to a further aspect there is provided a system for communicating data relevant to a freshness of juice stored in a portable container, the system comprising: an optical sensor arranged to sense particles in the juice stored in the portable container; and a processing system configured to: receive optical data from the optical sensor; determine the data relevant to the freshness of said juice stored in the portable container, the determining the data relevant to the freshness of the juice stored in the portable container comprising extracting a measure of particle agglomeration in the juice from the optical data; and communicate said data to a user of the portable container.

Communicating the data relevant to the freshness of the juice stored in the portable container may, for instance, be by the processing system being configured to control a user interface, e.g. a user interface included in a portable device, to communicate the data, as previously described.

In some embodiments, the optical sensor is mountable on and/or in the portable container.

In some embodiments, the measure of particle agglomeration comprises a measure of change in one or more parameters of a particle size distribution over a storage period.

In some embodiments, the optical data comprises initial optical data obtained at a time of the juice being initially stored in the portable container, and subsequent optical data obtained after one or more time periods from said time of the juice being initially stored. In such embodiments, the determining the data relevant to the freshness of the juice stored in the portable container comprises extracting the measure of particle agglomeration by comparison of the initial optical data and the subsequent optical data.

The one or more time periods may each have a predetermined duration.

In some embodiments, the time of the juice being initially stored is based on a user entry entered by the user via a user interface.

Such a user interface may be included a portable device, for example a smartphone and/or tablet computer.

As well as enabling the user to enter the user entry, such a portable device may, in some embodiments, also include a user interface controllable by the processing system to communicate the data relevant to the freshness of said juice stored in the portable container.

In other embodiments, the initial storage of the juice in the container is detected, for example by the optical sensor, such that the user is not required to enter the user entry.

In some embodiments, the processing system is configured to control the user interface to provide instructions for making the juice, with the instructions comprising a prompt for the user to enter said user entry.

More generally, embodiments described herein in relation to the system may be applicable to the method and computer program, and embodiments described herein in relation to the method or computer program may be applicable to the system.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
FIG. 1 provides a flowchart of a method according to an example;
FIG. 2 provides a block diagram of a system according to an example;
FIG. 3 schematically depicts a portable container for storing juice according to an example;
FIG. 4 shows a magnified image of juice particle agglomerates;
FIG. 5 provides magnified images showing juice particle agglomeration over time;
FIG. 6 shows a workflow involving juice preparation and storage according to an example; and
FIG. 7 provides a flowchart including steps of the method shown in FIG. 1.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Provided is a method for communicating data relevant to a freshness of juice stored in a portable container. The method comprises receiving optical data from an optical sensor arranged to sense particles in the juice stored in the portable container. The determination of the data relevant to the freshness of the juice stored in the portable container comprises extracting a measure of particle agglomeration in the juice from the optical data. The thus determined data is then communicated to a user of the portable container. Further provided is a computer program for implementing the method, and a related system for communicating the data relevant to the freshness of the juice stored in the portable container.

Juice extracted from fruit and/or vegetables may be a two phase colloidal system with a dispersed solid phase called a cloud including particles, such as fiber particles, and a continuous liquid phase called serum, in other words clear juice. Over time, such particles may cluster together so as to form an aggregate or agglomerate. The term "agglomeration" may refer to formation of particle assemblages in a suspension, leading to destabilization of the colloidal system. The formation of such aggregates/agglomerates may increase the rate of sedimentation and cause more rapid loss of stability, eventually resulting in relatively large particle assemblages settling as a precipitate at the bottom of the juice.

Even in well-filtered, clear juice in which particles are relatively small, for example less than 0.1 µm, the particles may agglomerate over time, for example due to aggregation of dietary fibre, tannins and starches, so that the juice becomes progressively less clear.

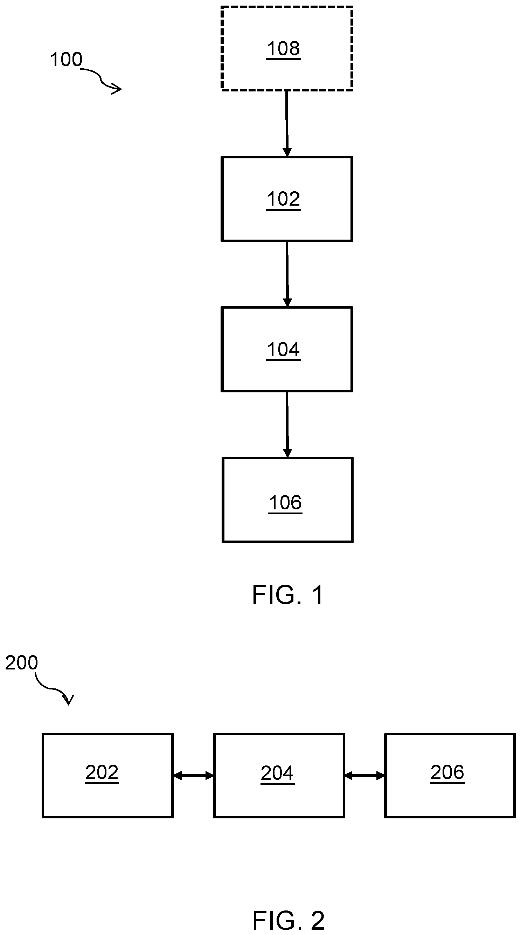

FIG. 1 shows a flowchart of a method 100 according to an example. The method 100 comprises receiving 102 optical data from an optical sensor arranged to sense particles in juice stored in a portable container. The method 100 further comprises determining 104 data relevant to a freshness of the juice stored in the portable container. This determination 104 comprises extracting a measure of particle agglomeration in the juice from the optical data.

The present disclosure is based, at least in part, on the insight that the agglomeration behavior of particles in juice, for example juice extracted from fruit and/or vegetables, can be used as a convenient freshness indicator of the juice when stored in a portable container. This is because the timescale of the agglomeration may align with the timescale of decomposition processes, for example oxidation, associated with declining freshness. Moreover, a measure of particle agglomeration in the stored juice can be conveniently extracted from the optical data received from the optical sensor arranged to sense particles in the juice stored in the portable container. Such an optical sensor can, for instance, be mounted in and/or on the portable container. The optical sensor will be described in more detail herein below.

The method 100 further comprises communicating 106 the data relevant to the freshness of the juice stored in the portable container to a user. Thus, the user is kept informed of the freshness of the stored juice.

FIG. 2 provides a block diagram of a system 200 according to an example. The system 200 comprises a processing system 204 configured to receive 102 optical data from an optical sensor 202; determine 104 data relevant to the freshness of said juice stored in a portable container, with this determination 104 comprising extracting a measure of particle agglomeration in the juice from the optical data; and communicate 106 the data to a user of the portable container. Thus, the processing system 204 of the system 200 may be configured to implement the method 100.

In at least some embodiments, such as that shown in FIG. 2, the system 200 comprises the optical sensor 202 together with the processing system 204. In other examples, the optical sensor 202 is provided, for example supplied to the user, separately from the processing system 204.

In some embodiments, and still referring to FIG. 2, the processing system 204 is configured to communicate 106 the data relevant to the freshness of the juice stored in the portable container by controlling a user interface 206.

The user interface 206 may be provided, e.g. supplied to the user, together with the optical sensor 202 and the processing system 204, as shown in FIG. 2. Alternatively or additionally, such a user interface 206 may be included together with at least part of the processing system 204 in a portable device, such as a smartphone or tablet computer. Such a portable device may enable the user to receive the data relevant to the freshness of the stored juice while on the move, e.g. when on the move with the portable container.

Such a user interface 206 may include a display and/or a speaker, such that the data may be communicated visually and/or audibly.

The communicating 106 may, for example, comprise controlling a display of a portable device to display the data relevant to the freshness of the juice stored in the portable container.

The optical sensor 202 may, as an alternative to being included in the portable device, be a separate component mountable on and/or in the portable container.

The optical sensor 202 can, for instance, be a built-in component of the portable container.

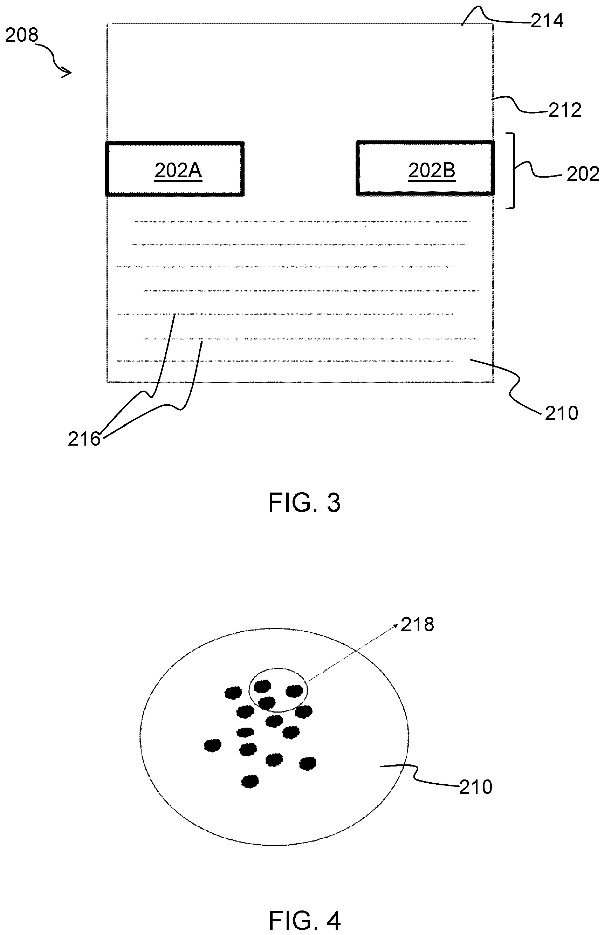

A portable container 208 in which juice 210 is being stored is schematically depicted in FIG. 3. In this example, the optical sensor 202 is mounted in, in other words inside, the portable container 208.

The optical sensor 202 is preferably mounted at an upper side of the portable container 208, so as to implement sensing, e.g. image capture, from above the juice 210 when the portable container 208 is uprightly orientated.

The optical sensor 202 may be mounted to, for example detachably mounted to, the portable container 208 in any suitable manner. Detachable mounting of the optical sensor 202 may, for instance, facilitating cleaning of the portable container 208 between uses because the optical sensor 202 can be detached prior to the cleaning, e.g. using a dishwasher.

Whilst not visible in FIG. 3, the optical sensor 202 may include a light source for illuminating the juice 210 stored in the portable container 208.

In such embodiments, the light source may assist in particle sensing, and thus help to improve the quality of the optical data from which the measure of particle agglomeration is extracted.

In some embodiments, such as that shown in FIG. 3, the optical sensor 202 is mounted to a sidewall 212 of the portable container 208. Alternatively or additionally, the optical sensor 202 may be mounted to a top 214, for example to an openable lid, of the portable container 208.

Any suitable optical sensor 202 can be used provided that the optical sensor 202 is capable of sensing particles 216 in the juice 210 and generating optical data from which the measure of particle agglomeration can be extracted.

In some embodiments, the optical sensor 202 comprises an image detection unit, for example a camera. Such an image detection unit may include one or more image sensors, for example a charge coupled device (CCD) image sensor or a complementary metal-oxide-semiconductor (CMOS) image sensor.

An image detection unit may provide image data, for example a characteristic of one or more particle agglomerates in the juice 210, from which the measure of particle agglomeration is extractable.

In the non-limiting example shown in FIG. 3, the optical sensor 202 comprises a pair of image sensors 202A, 202B arranged to sense the particles 216 in the juice 210. The measure of particle agglomeration is extractable from the image data generated by the image sensors 202A, 202B. In other examples, the optical sensor 202 includes a single image sensor.

In some embodiments, the image detection unit comprises an optical microscope that cooperates with the image sensor(s) 202A, 202B to allow microscope images to be captured.

In such embodiments, the image sensor(s) 202A, 202B and the optical microscope may be conveniently provided as an integrated unit or as separate units in which the image sensor(s) 202A, 202B is or are arranged to capture a microscope image of the juice 210 provided by the optical microscope.

In general, the optical sensor 202 may, via sensing of the particles 216 in the juice 210, generate the optical data in a first step, and an optical data processing module included in the processing system 204 may, in a second step, extract the measure of particle agglomeration in the stored juice 210 from the optical data.

The first step may comprise the image sensor(s) 202A, 202B capturing a real-time image of the juice 210, with the second step comprising (i) analyzing and extracting a particle size distribution using an algorithm, e.g. a backend algorithm, run on the processing system 204, and (ii) determining an average particle size from the particle size distribution.

The average particle size may, for instance, be a median particle size, in other words D₅₀, obtained from the particle size distribution.

More generally, and referring to FIG. 4, the measure of particle agglomeration may be extracted from the optical data, e.g. image data enabling assessment of particle assemblage(s) 218 in the juice 210, captured at a single point in time, or may be extracted from optical data, e.g. image data, captured at a plurality of points over a period time.

It is noted that FIG. 4 can be regarded as depicting a microscope image of the juice 210 that shows the particle assemblage(s) 218 in the juice 210.

As an example of extracting the measure of particle agglomeration from optical data captured at a single point in time, the average particle size, e.g. D₅₀, can be determined from an image of the juice 210 in the manner described above, a comparison of the thus determined average particle size with a look-up table can be made, with the comparison enabling extraction of the measure of particle agglomeration in the juice 210. The data relevant to the freshness of the juice 210 stored in the portable container 208 can then be communicated 106 to the user, for example immediately following the determination 104 of the data, e.g. in the form of a real-time message.

Table 1 provides an example of such a look-up table which provides, considering the food variety, four key cut-off points for categorizing the freshness of the juice 210 stored in the portable container 208.

**Table 1**

| | Ave. value of particle size (µm) | Description |
|---|---|---|
| Level 0 | <109 | Fresh as 'just-made' & no detectable sediment |
| Level 1 | 109~150 | Drinkable & Detectable and Slight sediment. |
| Level 2 | 160~600 | Drinkable & Detectable and moderate sediment |
| Level 3 | >600 | Suggest not to drink |

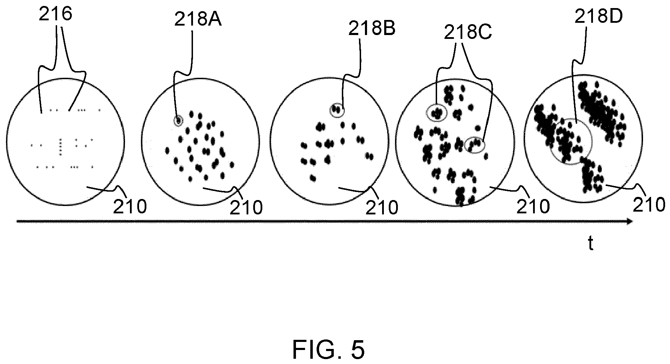

FIG. 5 provides magnified images showing juice particle agglomeration over time, t. The far left magnified image shows particles 216 present in the juice 210. As time passes, the particles 216 agglomerate into progressively larger particle assemblages 218A, 218B, 218C, 218D, leading to destabilization of the colloidal system.

The measured average particle size has been found to vary with the juice preservation period, in other words the time during which the juice 210 is stored in the portable container 208, e.g. at room temperature, as follows:

**Table 2**

| Juice Preservation Period/hour | Average Particle Size (µm) |
|---|---|
| 1 | <30 |
| 1.5 | 30~75 |
| 2 | 109~150 |
| 2.5 | 150~180 |
| 3 | 180~600 |

More severe sedimentation may occur after 3 hours, hence the method 100 of the present disclosure is preferably applicable for juice storage periods up to 3 hours, e.g. 3 up to 3 hours at room temperature.

In some embodiments, the measure of particle agglomeration comprises a measure of change in one or more parameters of a particle size distribution in the juice 210 over a juice storage period.

The particle agglomeration may cause a particle size distribution to change over a juice storage period, in other words as a function of time during which the juice 210 is stored in the portable container 208. This may be due to growing particle assemblages 218A, 218B, 218C, 218D, as shown in FIG. 5, increasing the relative amount of larger particles evident in the particle size distribution.

In some embodiments, the one or more parameters of the particle size distribution is or are selected from an average particle size, e.g. D₅₀, determined from the particle size distribution and a dispersity of the particle size distribution.

Following a change in such parameter(s) of the particle size distribution, for example the average, e.g. mean or median, particle size over time may enable a trend of aggregation and sedimentation of the stored drink to be established.

Such a trend can, for example, assist in terms of indicating how quickly the freshness of the juice 210 is deteriorating.

The measure of change in one or parameters of the particle size distribution over the juice storage period can, for example, be regarded as a pattern of particle size distribution. In such embodiments, the optical data processing module, e.g. in the form of an image processing module, included in the processing system 204 may be configured to extract the pattern of particle size distribution using an algorithm, e.g. a backend algorithm, run on the processing system 204.

In some embodiments, the optical data comprises initial optical data obtained by the optical sensor 202 at a time of the juice 210 being initially stored in the portable container 208, and subsequent optical data obtained by the optical sensor 202 after one or more time periods from the time of the juice 210 being initially stored. In such embodiments, the determining 104 the data relevant to the freshness of the juice 210 stored in the portable container 208 comprises extracting the measure of particle agglomeration by comparison of the initial optical data and the subsequent optical data.

Such a comparison between the initial optical data and the subsequent optical data may assist in terms of permitting the freshness of the juice 210 following the time period(s) to be compared to the freshness of the juice 210 upon being initially stored in the portable container 208. In this way, the measure of particle agglomeration can be made independent of the particle size in the asprepared juice 210.

In some embodiments, the time of the juice 210 being initially stored is based on a user entry entered by the user via a user interface, for example the user interface 206 included in the system 200 shown in FIG. 2. Such a user interface 206 may be included in a portable device, for example a smartphone and/or tablet computer, as previously described.

As well as enabling the user to enter the user entry, the user interface 206 of such a portable device may, in some embodiments, also be controllable by the processing system 204 to communicate the data relevant to the freshness of the juice 210 stored in the portable container 208.

In other embodiments, the initial storage of the juice 210 in the portable container 208 is detected, for example by the optical sensor 202, such that the user is not required to enter the user entry in order to register the time of the juice 210 being initially stored in the portable container 208.

In some embodiments, the processing system 204 is configured to control the user interface 206 to provide instructions for making the juice 210, with the instructions comprising a prompt for the user to enter said user entry.

In such embodiments, the processing system 204 can be regarded as including a drink processing unit configured to enable the user to select a juice recipe according to their individualized needs.

It is noted that providing the instructions for making the juice 210 can be implemented with or without connection to a portable device, e.g. smartphone. In embodiments, in which the instructions are provided via the user interface 206 of a portable device, such as a smartphone or tablet computer, a dedicated application can guide the user as to how to make the drink by providing step by step instructions. The application may send a reminder message to the user to check if they would like to preserve part or all of the prepared drink. If the user answers in the affirmative to the preserving/storing question, the application may suggest that they store the juice 210 in the portable container 208, e.g. "preservation cup/jar", for freshness monitoring.

The portable container 208 may be designed so that the user can bring the portable container 208 containing the juice 210 with them when leaving their house.

In some embodiments, the one or more time periods each have a predetermined duration. Thus, the measure of particle agglomeration can be obtained at one or more defined points in time during storage of the juice 210. This can assist with freshness monitoring.

In some embodiments, and referring again to FIG. 1, the method 100 comprises controlling 108 the optical sensor 202 to obtain the subsequent optical data after the one or more time periods from the time of the juice 210 being initially stored.

The processing system 204 may thus be configured to control 108 the optical sensor 202 to obtain the subsequent optical data after the one or more time periods from the time of the juice 210 being initially stored.

The controlling 108 the optical sensor 202 to obtain the subsequent optical data may accordingly be implemented automatically. This may assist to alleviate the burden on the user to him/herself take steps to monitor the freshness of the juice 210 stored in the portable container 208.

Alternatively or additionally, the user may him/herself control the optical sensor 202 to obtain the subsequent optical data, for example in response to a prompt provided via the user interface 206, or unprompted, e.g. when considering whether to drink the juice 210 stored in the portable container 208.

In some embodiments, the data relevant to the freshness of the juice 210 stored in the portable container 208 comprises a sediment status indicator indicative of a degree of sedimentation in the juice 210.

In such embodiments, the processing system 204 can be regarded as including a separation indicator unit.

The determining 104 the data relevant to the freshness of the stored juice 210 may correspondingly include determining the sediment status indicator based on the measure of agglomeration extracted from the optical data.

In some embodiments, the sediment status indicator is a sediment level selected from a plurality of pre-set levels.

By way of a non-limiting example, such a plurality of levels may include or be defined by the following: Level 0: fresh as 'just-made' and no detectable sediment; Level 1: drinkable but slight sedimentation detected; Level 2: drinkable but moderate sedimentation detected; and Level 3: suggest not to drink.

In such an example, and referring also to Table 1 above, Level 0 may be based on an average particle size in the stored juice 210 being <109 µm; Level 1 may be based on an average particle size in the stored juice 210 being 110-159 µm; Level 2 may be based on an average particle size in the stored juice 210 being 160-600 µm; and Level 3 may be based on an average particle size in the stored juice 210 being >600 µm.

More generally, the data relevant to the freshness of the juice 210 stored in the portable container 208 may be communicated 106, for example as a report and/or reminder message to the user, to enable the user to decide whether or not to drink the stored juice 210.

Such a report and/or reminder may be provided visually and/or audibly, for example via a user interface 206, e.g. display and/or loudspeaker, included in the above-described portable device.

In some embodiments, the data relevant to the freshness of the juice 210 stored in the portable container 208 comprises a reminder for the user to consume the juice 210 and/or an estimated time left for the user to consume the juice 210.

Thus, the determining 104 may include determining the reminder and/or the estimated time left for the user to consume the juice 210 based on the measure of agglomeration extracted from the optical data.

Such a reminder and/or estimated time left may assist the user to consume the stored juice 210 while it is still sufficiently fresh.

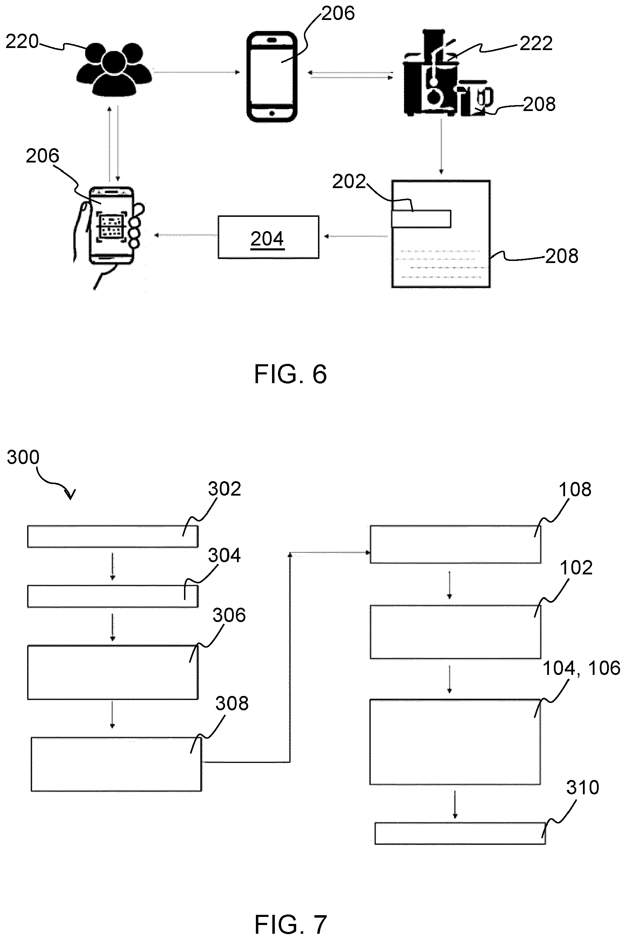

FIG. 6 shows a workflow involving juice preparation and storage according to an example. In this example, the user 220 may receive instructions for making juice via the user interface 206 of a portable device, for example a smartphone or tablet computer. The user 220 may operate a juicer or blender 222 according to the instructions to prepare the juice 210. Once prepared, the juice 210 is stored in the portable container 208, as shown.

The instructions may prompt the user 220 to, upon storing the juice 210 in the portable container 208, enter the user entry to register the time of the juice 210 being initially stored in the portable container 208, as previously described.

The data relevant to the freshness of the juice 210 stored in the portable container 208 is determined 104 by the processing system 204 extracting the measure of particle agglomeration in the juice 210 from the optical data received from the optical sensor 202 arranged to sense particles in the stored juice 210, as previously described.

The processing system 204 then controls the user interface 206 to communicate 106 the data relevant to the freshness of the stored juice 210, for instance by communicating 106 one of the above-mentioned sediment levels (Level 0: fresh as 'just-made' and no detectable sediment; Level 1: drinkable but slight sedimentation detected; Level 2: drinkable but moderate sedimentation detected; and Level 3: suggest not to drink), e.g. together with the juice preservation period, e.g. a number of hours of storage in the portable container 208.

On the basis of the communicated data, the user 220 may opt to drink or discard the juice 210 stored in the portable container 208.

FIG. 7 provides a flowchart of a method 300 including steps of the method 100 shown in FIG. 1. In step 302, the user 220 may select a recipe from a list of recipes. In step 304, the user 220 may prepare the drink according to the selected recipe. The prepared drink is stored in the portable container 208, e.g. in a "preservation jar/container", in step 306. In step 308, the user 220 decides to leave the portable container 208 at home or take it with them when leaving their home.

In step 108, the optical sensor 202, e.g. image detection unit, is controlled to take images at predetermined periods.

In step 102, the image data is received by the processing system 204, and in the block denoted by reference numerals 104, 106, the measure of particle agglomeration is extracted from the image data by determining the average particle size, the determined average particle size is compared to the above-mentioned look-up table to determine 104 the data relevant to the freshness of the stored juice 210, and the user interface 206 is controlled to communicate 106 the data.

The data may be communicated 106 in the form of reminder messages and/or feedback relating to the freshness of the stored juice 210, e.g. via a mobile application running on a portable device and/or via a user interface 206 included in a different device, for instance a user interface 206 included in the portable container 208 itself.

In step 310, the user 220 decides whether to drink the stored juice 210 or not, on the basis of the data communicated to the user 220.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by the processing system 204 may be implemented by a single processor or by multiple separate processing units which may together be considered to constitute a "processing system". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A method (100) for communicating data relevant to a freshness of juice stored in a portable container, the method comprising:
receiving (102) optical data from an optical sensor arranged to sense particles in the juice stored in the portable container;
determining (104) said data relevant to the freshness of said juice stored in the portable container, wherein said determining comprises extracting a measure of particle agglomeration in the juice from the optical data; and
communicating (106) said data to a user of the portable container.

2. The method (100) according to claim 1, wherein the measure of particle agglomeration comprises a measure of change in one or more parameters of a particle size distribution over a juice storage period.

3. The method (100) according to claim 1 or claim 2, wherein the optical data comprises initial optical data obtained by the optical sensor at a time of the juice being initially stored in the portable container, and subsequent optical data obtained by the optical sensor after one or more time periods from said time of the juice being initially stored, said determining (104) comprising extracting the measure of particle agglomeration by comparison of the initial optical data and the subsequent optical data; optionally wherein the one or more time periods each have a predetermined duration.

4. The method (100) according to claim 3, comprising controlling (108) the optical sensor to obtain the subsequent optical data after said one or more time periods from said time of the juice being initially stored.

5. The method (100) according to any of claims 1 to 4, wherein the data comprises a sediment status indicator indicative of a degree of sedimentation in the juice.

6. The method (100) according to any of claims 1 to 5, wherein the communicated data comprises a reminder for the user to consume the juice and/or an estimated time left for the user to consume the juice.

7. The method (100) according to any of claims 1 to 6, wherein the communicating (106) comprises controlling a user interface to communicate the data.

8. The method (100) according to any of claims 1 to 7, wherein the communicating (106) comprises controlling a display of a portable device to display the data.

9. A computer program product comprising computer program code which, when executed on a computing device having a processing system, causes the processing system to perform the method (100) according to any of claims 1 to 8.

10. A system (200) for communicating data relevant to a freshness of juice stored in a portable container (208), the system comprising:
an optical sensor (202) arranged to sense particles in the juice stored in the portable container; and
a processing system (204) configured to:
receive optical data from the optical sensor;
determine said data relevant to the freshness of said juice stored in the portable container, said determining comprising extracting a measure of particle agglomeration in the juice from the optical data; and
communicate said data to a user of the portable container.

11. The system (200) according to claim 10, wherein the optical sensor (202) is mountable on and/or in the portable container (208).

12. The system (200) according to claim 10 or claim 11, wherein the measure of particle agglomeration comprises a measure of change in one or more parameters of a particle size distribution over a storage period.

13. The system (200) according to any of claims 10 to 12, wherein the optical data comprises initial optical data obtained at a time of the juice being initially stored in the portable container, and subsequent optical data obtained after one or more time periods from said time of the juice being initially stored, said determining comprising extracting the measure of particle agglomeration by comparison of the initial optical data and the subsequent optical data; optionally wherein the one or more time periods each have a predetermined duration.

14. The system (200) according to claim 13, wherein the time of the juice being initially stored is based on a user entry entered by the user via a user interface (206).

15. The system (200) according to claim 14, wherein the processing system (204) is configured to control the user interface (206) to provide instructions for making the juice, said instructions comprising a prompt for the user to enter said user entry.
